# EUROPEAN PATENT SPECIFICATION

(11) **EP 0 786 011 B1**
(45) Date of publication and mention of the grant of the patent: **05.04.2000**
(21) Application number: 95935926.6
(22) Date of filing: 10.10.1995
(51) Int. Cl.: C12P 33/02, C12P 33/06, C07J 73/00

(54) **Microbiological process for the preparation of 1-unsaturated 17-beta-carboxysubstituted 3-oxo-4-azaandrostan-3-ones**
Mikrobiologisches Verfahren zur Herstellung von 1-ungesättigten, 17-beta-carboxysubstituierten 3-Oxo-4-Azaandrostan-3-onen
Procédé microbiologique de préparation de 3-oxo-4-azaandrostan-3-ones carboxysubstituées en position 17 et comportant une double liaison en position 1

(30) Priority: 13.10.1994 IT MI942094
(43) Date of publication of application: 30.07.1997
(60) Divisional application: 98124126.8
(73) Proprietor: POLI INDUSTRIA CHIMICA S.p.A., 20121 Milano (IT)
(72) Inventor: POLI, Stefano, I-20089 Quinto de' Stampi - Rozzano (IT); MAGNI, Ambrogio, I-20089 Quinto de' Stampi - Rozzano (IT); GRISENTI, Paride, I-20089 Quinto de' Stampi - Rozzano (IT); LEONI, Massimo, I-20089 Quinto de' Stampi - Rozzano (IT)
(74) Representative: Minoja, Fabrizio, Dr.
(86) International application number: EP9503992
(87) International publication number: WO9612034

(56) References cited:
- EP-A- 0 599 376
- EP-B- 0 350 488
- DE-B- 1 070 176
- US-A- 2 969 304
- THE JOURNAL OF THE AMERICAN CHEMICAL SOCIETY, vol. 82, July-September 1960 R.M. DODSON et al. "Microbiological Transformations. V. 1alpha- and 2beta-Hydroxylations of C19-Steroids", pages 4026-4033 (cited in the application).

## Description

The present invention relates to a microbiological process for the transformation of 17β-substituted 4-azaandrostan-3-ones.

The class of molecules known as 4-azasteroids are known to be capable of inhibiting specifically the process of 5a-reduction of testosterone. Up to now, a number of examples of inhibitors of the enzyme 5a-reductase having such a structure are known in literature (Rasmusson, G, H.; Reynolds, G.F.; Utne T.; Jobson R.B.; Primka R.L.; Broocks J. R.; Berman C. J. Med. Chem. 1984, 27, 1690-1701. Rasmusson, G, H.; Reynolds, G. F.; Steimberg, N. G.; Walton, B.; Patel G.; F.; Liang T.; Cacsieri M. A.; Cheung A. H.; Broocks J. R.; Berman C. J. Med. Chem. 1986, 29, 2298-2315; BP 155096, BP 538192, EP468012, BP 484094). Many of the molecules studied from such a point of view have the common feature of having one unsaturation in position 1, as illustrated in the following Formula 1: 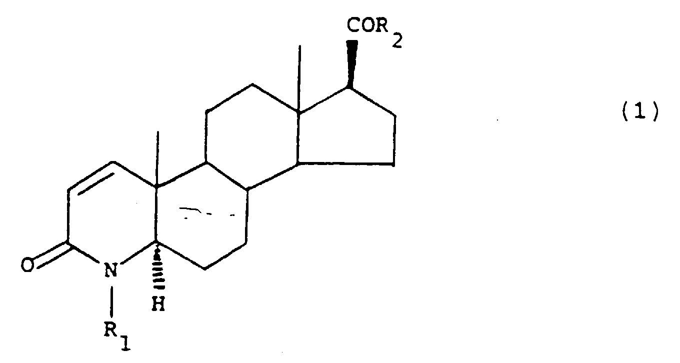 wherein R₁ is hydrogen or a lower alkyl group, R₂ is a OH, OR₃, NHR₃ group, wherein R₃ is a straight or branched C₁-C₉ alkyl group, cycloalkyl optionally containing one o more heteroatoms selected from S, 0, N, C₁₀-C₂₀ polycycloalkyl, aryl.

The procedures described for the introduction of this unsaturation are numerous: in some cases, phenylselenic anhydride is used as the reactive (Back T.G. J. Org. Chem. 1981, 46, 1442-1446), or said compounds can be prepared either through a sulfenylation reaction (Zoretic P.A. et al. J. Org, Chem. 1978, 43, 1379-1382) or, as reported more recently, through a silylation and oxidation process, via the formation of an adduct with DDQ (2,3-dichloro-5,6-dicyano-1,4-benzoquinone). All these procedures require drastic experimental conditions (for example, the reaction temperatures vary, depending on the procedure followed, from -78° to +120°C, specific cautions must be taken for the used solvents, which should be dry, the used reactives are remarkably toxic). Interestingly, though the conversion percentages into the desired product are in some cases satisfactory, the used reactives are markedly coloured and not atoxic, which makes considerably complex the purification procedures to obtain a pharmaceutically acceptable product, to the detriment of the actual yield of the process. Moreover, the environmental problem of the disposal of the reactives used on an industrial scale asks for novel, less polluting methods to carry out this chemical step.

Some types of microorganisms are known from literature to be capable of transforming the traditional steroid moiety (Phytochemistry, 1984, 23(10), 2131-2154; Microbial transformation of steroids and alkaloids, Iizuka H., Naito A. University of Tokyo Press, 1967; Yamanè T. et al. Biotechnology and Bioengineering, 22, 1979, 2133-2141; Sih C.J. et al. Biochem. Biophys. Acta 38, 1969, 378-379; Dodson R.M. et al. J. Am. Chem. Soc. 82, 1960, 4026) but we found no evidences from literature of any type of biotransformation on structures such as 17β-carboxy substituted 4-azaandrostan-3-ones as described in formula 1.

Surprisingly, it has been found that some microorganisms are capable of biotransforming the azasteroidal moiety so as to introduce an unsaturation into position 1. Thus, using, for example, the known 17-N-t-butylcarbamoyl-4-azaandrostan-3-one as the substrate (Reynolds, G. F.; Steimberg, N. G.; Walton, E.; Patel G. F.; Liang T. Cacsieri M. A.; Cheung A. H.; Broocks J. R.; Berman C. J. Med. Chem. 1986, 29, 2298-2315) and Arthrobacter simplex, Nocardia sp. as the microorganisms, we successfully obtained the corresponding derivative dehydrogenated at position 1, in yields ranging from 20 to 80% (Scheme 1).

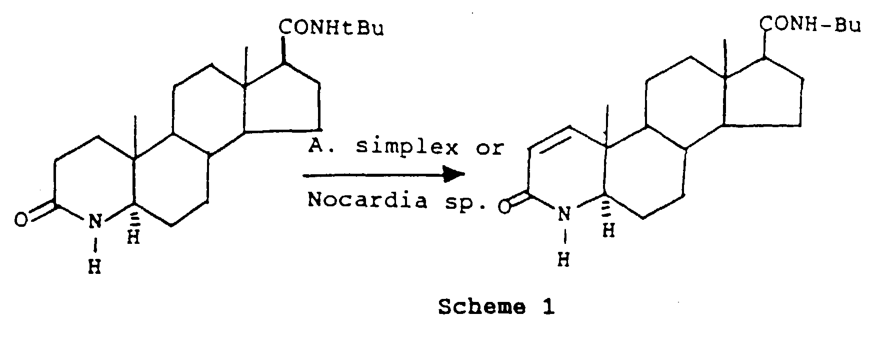

Therefore, an object of the present invention is a process for the preparation of compounds of formula (1): 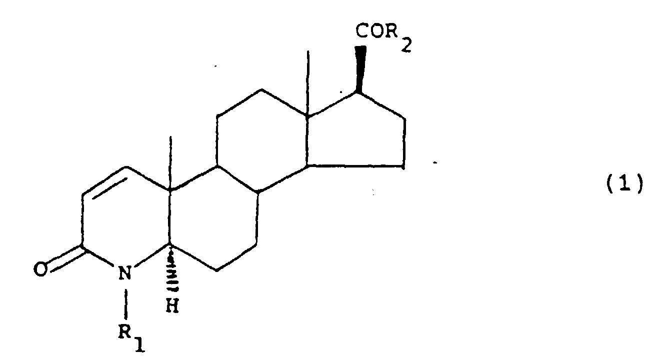 wherein R₁ is hydrogen or a lower alkyl group, R₂ is a OH, OR₃, NHR₃ group, wherein R₃ is a straight or branched C₁-C₉ alkyl group, cycloalkyl containing optionally one or more heteroatoms selected from S, 0, N, C₁₀-C₂₀ polycycloalkyl, aryl; which process comprises the steps of:
a) culturing a microorganism of the genus Arthrobacter or Nocardia in a suitable culture medium;
b) inducing the production of Δ1 dehydrogenase in said microorganism;
c) separating the cells of said microorganism;
d) incubating said microorganism with a compound of formula (2): 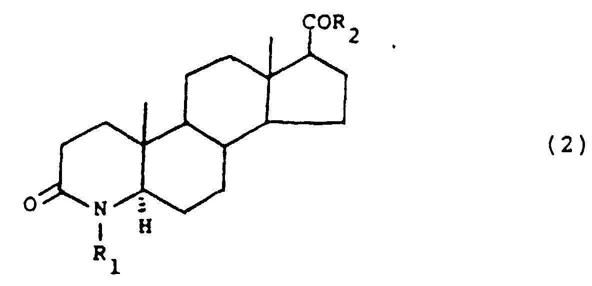 wherein R₁ and R₂ are as defined above;
e) recovering the biotransformed product;
f) optionally alkylating the steroid nitrogen atom.

Following a general procedure, bacteria belonging to the genera Arthrobacter or Nocardia are cultured in suitable liquid media containing a carbon source, generally consisting of an hexose, usually glucose, in concentrations ranging from 0.5 to 15 g/l, preferably from 1 to 10 g/l, and of a mixture of complex substances such as peptones, hydrolysates and extracts, for example meat, blood, gelatin peptones; casein or albumin hydrolysates; yeast extracts or autolysates, in a total concentration ranging from 10 to 100 g/l, preferably from 20 to 50 g/l. The culture is carried out at temperatures from 25 to 35°C, preferably at 27-29'C, for a time ranging from 20 to 72 hours (usually 24 hours). After 5-12 hours from the inocule, the culture broth is added with a solution or suspension containing a molecule capable of inducing the production of the enzyme Δ1 dehydrogenase in bacteria. For this purpose, steroid inducers such as progesterone or hydrocortisone can be used in concentrations ranging from 10 mg to 1 g/l (preferably from 100 to 500 mg/l). At the end of the culture, the cells are separated from the liquid by filtration or centrifugation and used. Either the cells in toto or those fractions having an interesting enzymatic activity can be used. It is also possible to keep the cell activity intact for some months, freezing the cell paste at -25°C. The transformation reaction is carried out in a buffer at pH 6.00-8.00 (preferably at pH 7.00) and at a temperature from 25 to 35°C, for times between 10 and 120 hours. To obtain a good conversion percentage, it is essential to use a buffer previously saturated with an organic solvent such as CHCl₃, tetradecanol, ethyl acetate, cyclohexane, benzene, n-butanol, and preferably to select from said solvents those with log P higher than 1.5, such as CHCl₃, to a maximum of 10% v/v. The resulting incubation medium has to be added with an external co-factor such as menadione or phenazine methasulfate, preferably menadione in concentrations ranging from 20 to 250 mg/l. The substrate to transform is added to the reaction mixture in concentrations ranging from 0.1 to 1 g/l, the substrate being added in a single addition, fractionally or continuously.

The reaction is then worked up filtering off the mycelium on Celite and extracting the filtrate with CHCl₃. The resulting organic extracts are dried over sodium sulfate, filtered and evaporated under vacuum to give a crude which is subsequently purified by chromatography on silica: by elution with dichloromethane/methanol 95/5, the dehydrogenated product is recovered in yields ranging from 20 to 80%, and by further isocratic elution, the not biotransformed product is recovered in yields ranging from 60 to 10%.

The dehydrogenation process described can also be carried out using the above mentioned microorganisms immobilized or using crude homogenates obtained from said microorganisms. The related experimental techniques are easy to carry out and known to those skilled in the art (Glass T.L. et al. J. Lipid. Res., 1982, 23, 352; Fukui S. et al. Acta Biotechnol. 1981, 1, 339; Omata T. et al. Eur. J. Microbiol. Biotechnol. 1979, 8, 143; Kim M.N. et al. Biotechnol. Letters 1982, 4, 233; Ohloson S. et al. Eur. J. Appl. Microbiol. Biotechnol, 1979, 7, 103). Moreover, the above mentioned reactions can also be effected using purified enzymes from the same microorganisms. In this case also, the possibility of recovering these dehydrogenase and using them as biocatalysts in the native state or supported over of a polymer matrix is widely described in literature (Grunwald J. et al. J. Am. Chem. Soc. 1986,108, 6732; Snjider-Kambers A.M. et al. Recl. Trav. Chim. Pays-Bas 1991, 110, 226; Santaniello E. et al. Chem. Rev. 92, 1992, 1071-1140).

The following examples further illustrate the invention.

### Example 1

### Dehydrogenation of 17-t-butylcarbamoyl-4-azaandrostan-3-one with A. simplex

The microorganism is kept on slants containing 9 ml of a medium having the following composition:

| Ingredients | Concentration (g/l) |
|---|---|
| Meat extract (Difco) | 1.500 |
| Yeast extract (Difco) | 3.000 |
| Peptone (Difco) | 6.000 |
| Casein hydrolysate (Costantino) | 4.000 |
| Dextrose (Cerestar) | 1.000 |
| Agar (Difco) | 18.000 |

The growth takes place in 18-20 hours at a temperature of 28°C. Transplants are carried out every 20-30 days. For the vegetative culture, 300 ml flasks are prepared containing 50 ml each of a medium with the following composition:

| Ingredients | Concentration (g/l) |
|---|---|
| Dextrose (Cerestar) | 1.000 |
| Bacteriologic peptone (Costantino) | 6.000 |
| Casein hydrolysate (Costantino) | 4.000 |
| Yeast autolysate (Costantino) | 3.000 |

pH (6.10-6.30) is adjusted to 7.00 by means of 1N NaOH and the medium is sterilised at 121°C for 15 minutes. The inocule is effected with 0.50 ml/flask of a suspension obtained washing a slant with 5 ml of saline solution. The flasks are then incubated at 28°C on a rotary shaker at 200 rpm for 17 hours. The subsequent step is carried out in a fermenter of 3 1 total volume, containing 1.7 1 of the above indicated medium and inoculated at the rate of 6% (120 ml). The operative conditions are as follows: 28°C, aeration 0.5 vvm at 200-500 rpm. After 6 hours, 1.7 ml of a suspension of hydrocortisone in 2 ml of acetone are added (at a temperature of 60°C) and culture is carried out for a further 18 hours. After 24 total hours, the cells are recovered by centrifugation at 4.800 xg (20 minutes) and suspended again in 0.1 M phosphate buffer, pH 7.00, saturated with CHCl₃ at the rate of 50 g/l humid weight. The cell suspension is distributed into 300 ml flasks (20 ml/flask), each of them being added with 0.125 ml of a solution prepared from 84 mg of 17β-t-butylcarbamoyl-4-azaandrostan-3-one and 8.7 mg of menadione in 2 ml of methanol (substrate final concentration 260 mg/l). The cultures are incubated at a temperature of 30°C for 72 hours over rotary shaker at 250 rpm. The reaction is then worked up filtering the reaction broth on celite and extracting the filtrate with CHCl₃ (1:1 v/v to the aqueous phase). The organic extracts are dried over sodium sulfate, filtered and evaporated to dryness to give a crude which is subsequently purified by chromatography over silica: gradient elution with CH₂Cl₂/MeOH 95/5 allows to recover the desired product in 60% yields and the starting product in 20% yields. ¹H-NMR (60 MHz): 0.70 (s, 3H), 0.97 (s, 3H), 1.40 (s, 9H), 3.1-3.6 (m, 1H), 5.2 (m, 1H); 6.3 (m, 1H), 5.8 (d, J = 9Hz, 1H), 6.7 (d, J = 9Hz, 1H).
M.p. 253-254°C.

## Claims

1. A process for the preparation of compounds of formula (1): 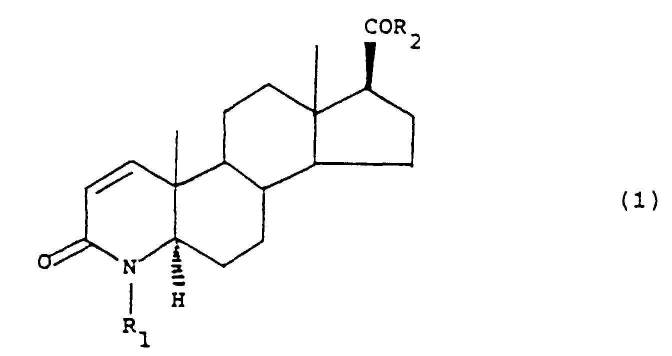 wherein R₁ is hydrogen or a lower alkyl group, R₂ is a OH, OR₃, NHR₃ group, wherein R₃ is a straight or branched C₁-C₉ alkyl group, cycloalkyl containing optionally one or more heteroatoms selected from S, O, N, C₁₀-C₂₀ polycycloalkyl, aryl; which process comprises the steps of:
a) culturing a microorganism of the genus Arthrobacter or Nocardia in a suitable culture medium;
b) inducing the production of Δ1 dehydrogenase in said microorganism;
c) separating the cells of said microorganism;
d) incubating said microorganism with a compound of formula (2): 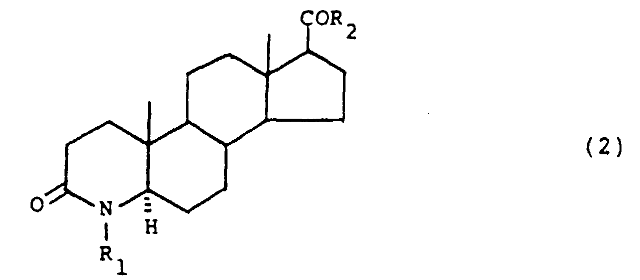 wherein R₁ and R₂ are as defined above;
e) recovering the biotransformed product;
f) optionally alkylating the steroid nitrogen atom.

2. A process according to claim 1, wherein the microorganism is Arthrobacter simplex or Nocardia sp..

3. A process according to claims 1-2, wherein step b) is carried out by means of steroid-type inducers.

4. A process according to claim 3, wherein said inducer is hydrocortisone.

5. A process according to claims 1-4, wherein step d) is effected in a buffer at pH from 6 to 8 previously saturated with a solvent having log P higher than 1.5, until a maximum of 10% v/v.

6. A process according to claim 5, wherein said solvent is chloroform.

7. A process according to claims 5 and 6, wherein said buffer is added with menadione or phenazine metasulfate.

8. A process according to claims 1-7, wherein in compound of formula 1 R₂ is a tert-C₄H₉-NH- group.

## Patentansprüche

1. Verfahren zur Herstellung von Verbindungen der Formel (1): 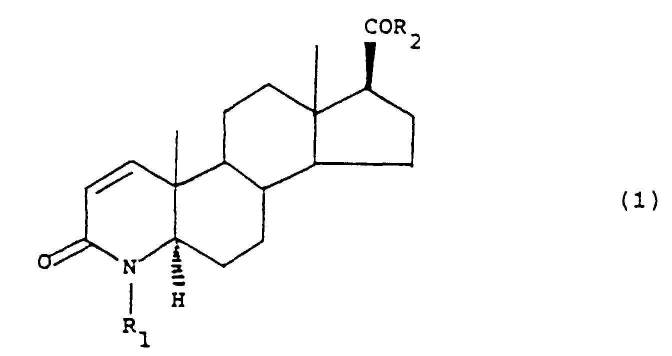 worin R₁ Wasserstoff oder eine niedrige Alkylgruppe ist, R₂ eine OH-, OR₃-, NHR₃-Gruppe ist, worin R₃ eine gerade oder verzweigte C₁-C₉-Alkylgruppe, Cycloalkyl, welches wahlweise eines oder mehrere Heteroatome enthält, ausgewählt von S, O, N, C₁₀-C₂₀ Polycycloalkyl, Aryl ist; wobei das Verfahren besteht aus den Stufen:
a) Züchtung eines Mikroorganismus des Genus Arthrobacter oder Nocardia in einem geeignetem Züchtungsmedium;
b) Induzierung der Produktion von Δ1 Dehydrogenase in dem Mikroorganismus;
c) Trennung der Zellen des Mikroorganismus;
d) Inkubation des Mikroorganismus mit einer Verbindung der Formel (2) : 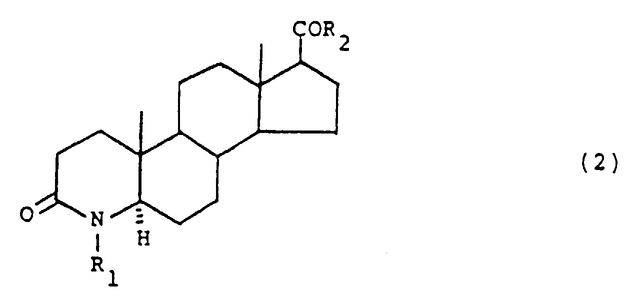 worin R₁ und R₂ wie vorstehend definiert sind;
e) Wiedergewinnung des biotransformierten Produkts;
f) wahlweise Alkylierung des steroiden Stickstoffatoms.

2. Verfahren nach Anspruch 1, bei welchen der Mikroorganismus Arthrobacter simplex oder Nocardia sp. ist.

3. Verfahren nach den Ansprüchen 1 und 2, bei welchem die Stufe b) mittels Induktoren des stereoiden Typs durchgeführt wird.

4. Verfahren nach Anspruch 3, bei welchem der Induktor Hydrocortison ist.

5. Verfahren nach den Ansprüchen 1 bis 4, bei welchem die Stufe d) in einer Pufferlösung mit einem pH-Wert von 6 bis 8 bewirkt wird, die zuvor mit einem Lösungsmittel mit log P höher als 1.5 gesättigt wurde bis zu einem Maximum von 10% v/v.

6. Verfahren nach Anspruch 5, bei welchem das Lösungsmittel Chloroform ist.

7. Verfahren nach den Ansprüchen 5 und 6, bei welchem zu der Pufferlösung Menadion oder Phenazinmetasulfat hinzugefügt ist.

8. Verfahren nach den Ansprüchen 1 bis 7, bei welchem in der Verbindung der Formel (1) R₂ eine tertiäre C₄H₉-NH-Gruppe ist.

## Revendications

1. Un procédé pour la préparation de composés de formule (1) : 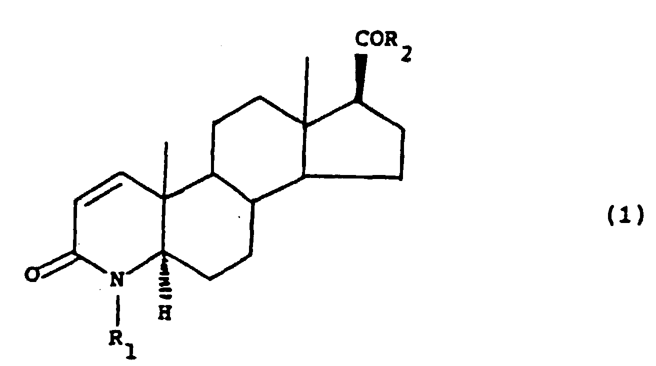 dans laquelle R₁ est l'hydrogène ou un groupe alkyle inférieur, R₂ est un groupe OH, OR₃, NHR₃, où R₃ est un groupe alkyle en C₁-C₉ linéaire ou ramifié, cycloalkyle contenant facultativement un ou plusieurs hétéroatomes choisis parmi S, O, N, polycycloalkyle en C₁₀-C₂₀, aryle ; ce procédé comprenant les étapes suivantes :
a) cultiver un microorganisme du genre *Arthrobacter* ou *Nocardia* dans un milieu de culture approprié ;
b) induire la production de Δ1-déshydrogénase dans ledit microorganisme ;
c) séparer les cellules dudit microorganisme ;
d) faire incuber ledit microorganisme avec un composé de formule (2) : 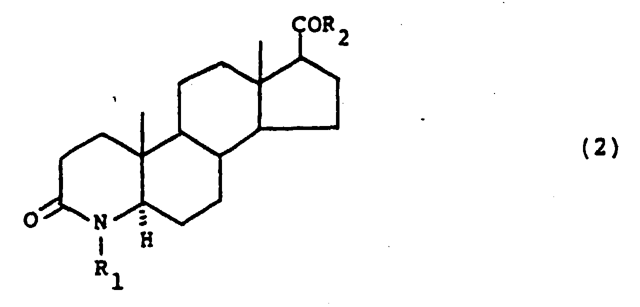 où R₁ et R₂ sont tels que définis ci-dessus ;
e) recueillir le produit biologiquement transformé ;
f) facultativement, alkyler l'atome d'azote du stéroïde.

2. Un procédé selon la revendication 1, dans lequel le microorganisme est *Arthrobacter simplex* ou *Nocardia sp.*

3. Un procédé selon les revendications 1 et 2, dans lequel l'étape b) est exécutée au moyen d'inducteurs du type stéroïde.

4. Un procédé selon la revendication 3, dans lequel ledit inducteur est l'hydrocortisone.

5. Un procédé selon les revendications 1 à 4, dans lequel l'étape d) est conduite dans un tampon à pH de 6 à 8 préalablement saturé avec un solvant ayant un log P supérieur à 1,5, jusqu'à un maximum de 10 % en volume.

6. Un procédé selon la revendication 5, dans lequel ledit solvant est le chloroforme.

7. Un procédé selon les revendications 5 et 6, dans lequel ledit tampon est ajouté avec de la ménadione ou du métasulfate de phénazine.

8. Un procédé selon les revendications 1 à 7, dans lequel, dans le composé de formule 1, R₂ est un groupe *tert*-C₄H₉-NH-.
